# EUROPÄISCHE PATENTSCHRIFT

(11) **EP 0 626 828 B1**
(45) Veröffentlichungstag und Bekanntmachung des Hinweises auf die Patenterteilung: **12.03.1997**
(21) Anmeldenummer: 94904179.2
(22) Anmeldetag: 22.12.1993
(51) Int. Cl.: A61B 17/60

(54) **SYSTEM ZUR OSTEOSYNTHESE AN DER WIRBELSÄULE, VERBINDUNGSELEMENT FÜR EIN SOLCHES SYSTEM UND WERKZEUG ZU DESSEN MONTAGE UND/ODER DEMONTAGE**
SYSTEM FOR OSTEOSYNTHESIS ALONG THE SPINAL COLUMN, CONNECTING ELEMENT FOR SUCH A SYSTEM AND TOOL FOR ASSEMBLING AND/OR DISMANTLING THE SAME
SYSTEME PERMETTANT DE PRATIQUER L'OSTEOSYNTHESE SUR LA COLONNE VERTEBRALE, ELEMENT DE RACCORDEMENT DUDIT SYSTEME ET OUTIL UTILISE POUR LE MONTER ET/OU LE DEMONTER

(30) Priorität: 23.12.1992 DE 4243951
(43) Veröffentlichungstag der Anmeldung: 07.12.1994
(73) Patentinhaber: PLUS ENDOPROTHETIK AG, 6343 Rotkreuz (CH)
(72) Erfinder: GRISS, Peter, D-35039 Marburg (DE)
(74) Vertreter: Popp, Eugen, Dr.
(86) Internationale Anmeldenummer: EP9303669
(87) Internationale Veröffentlichungsnummer: WO9414384

(56) Entgegenhaltungen:
- EP-A- 0 441 729
- EP-A- 0 487 895
- WO-A-89/09030
- WO-A-91/16020
- WO-A-93/11715
- CH-A- 335 245
- DE-C- 3 807 346
- DE-U- 9 310 668
- FR-A- 2 309 198
- FR-A- 2 682 280
- GB-A- 788 104
- US-A- 4 648 388
- US-A- 4 706 659

## Beschreibung

Die vorliegende Erfindung betrifft ein System zur Osteosynthese an der Wirbelsäule, insbesondere zur Stabilisierung von Wirbelsäulensegmenten, gemäß dem Oberbegriff des Anspruches 1.

Aus der EP 0 441 729 A1 ist ein derartiges System bekannt. Dieses weist mindestens ein stabartiges Verbindungselement und mindestens zwei jeweils an einem Wirbelsäulensegment verankerbare Haltemittel in Form von sogenannten Pedikelschrauben mit einem stimmgabelartigen Kopf auf, dessen beide Schenkel einen etwa U-förmigen Aufnahmeraum für das Verbindungselement begrenzen. Weiterhin umfaßt dieses bekannte System eine Klemmschraube, die zur Festlegung des Verbindungselements zwischen den beiden Schenkeln des stimmgabelartigen Schraubenkopfes in den Aufnahmeraum hineinschraubbar ist. Der Boden des Aufnahmeraums ist konkav schalenförmig ausgebildet in Zuordnung zu einem zwischen dem Boden des Aufnahmeraums und dem Verbindungselement angeordneten Schwenklagerelement, dessen dem Boden des Aufnahmeraums zugewandte Lagerfläche komplementär konvex gestaltet ist. Konkret ist das bekannte Schwenklagerelement als Kugelring mit Längsspalt ausgebildet, der vor Plazierung des Verbindungselements auf dasselbe aufgefädelt werden muß.

Daher hat sich der Erfinder die Aufgabe gestellt, das bekannte System derart weiterzuentwickeln, daß die Handhabung erheblich erleichtert und die Akzeptanz entsprechend erhöht wird.

Diese Aufgabe wird erfindungsgemäß durch die kennzeichnenden Merkmale des Anspruches 1 gelöst.

Der Kern der vorliegenden Erfindung liegt also darin, daß das dem stabartigen Verbindungselement zugeordnete Schwenklagerelement fester Bestandteil des an einem Wirbelsäulensegment verankerbaren Haltemittels ist, wobei der Freiheitsgrad für den Verbindungsstab beibehalten ist. Hinzu kommt die vereinfachte Handhabung sowie die geringere Gefahr, Teile des Systems versehentlich zu verlieren. Das erfindungsgemäße System erlaubt eine gleichbleibende und jederzeit reproduzierbare Operationsqualität. Aufgrund der konstruktiv einfachen Bauweise ist das erfindungsgemäße System für verschiedenste Indikationen verwendbar, welche eine Versteifung eines aus wenigstens zwei Wirbelsäulensegmenten bestehenden Wirbelsäulenabschnitts erfordern. Auch ist der Platzbedarf des hier beanspruchten Systems ebenso gering wie beim bekannten System, so daß eine hinreichende Deckung durch Muskel und Haut gewährleistet ist.

Vorteilhafte Ausgestaltungen der Erfindung sind in den Unteransprüchen beschrieben, wobei es gemäß Anspruch 2 im Rahmen der Erfindung liegt, daß die im Schwenklagerelement ausgebildete Nut zur Aufnahme des stabartigen Verbindungselements entsprechend dessen Querschnitt einen etwa halbkreisförmigen Querschnitt aufweist. Infolge der weitgehend übereinstimmenden Querschnitte von stabartigem Verbindungselement und Nut des Schwenklagerelements ist eine großflächige Auflage des stabartigen Verbindungselements zur Erzielung einer hohen Klemmwirkung ohne Erzeugung von Punktbeanspruchungen erreicht. Nachdem ein gerades bzw. geringfügig gebogenes stabförmiges Verbindungselement in der Nut des Schwenklagerelements aufgrund des aufeinander abgestimmten Querschnitts beliebig verschiebbar ist, läßt sich das Verbindungselement nach Anlage der Klemmschraube am Verbindungselement im Haltemittel noch beliebig in axialer Richtung verschieben und gleichzeitig um seine Längsachse rotieren. Folglich werden eine exakte Positionierung des gesamten Systems an den einzelnen Wirbelsäulensegmenten in dem entsprechenden Wirbelsäulenabschnitt ermöglicht und zugleich eine hohe Beanspruchung vor allem des Verbindungselements und damit zusammenhängend eine nur sehr geringe Lebensdauer des Implantates insgesamt vermieden. Dadurch, daß die Nut im Schwenklagerelement klemmschraubenseitig offen ist, läßt sich ohne Mühen das stabförmige Verbindungselement in diese Nut einlegen.

Zur weiteren Erhöhung der Klemmwirkung sind in die Oberfläche der Nut gemäß Anspruch 3 Querrippen, insbesondere ein Feingewinde, eingearbeitet.

Von besonderem Interesse für eine dauerhafte Halterung des stabartigen Verbindungselements in dem Haltemittel sind weiterhin die Merkmale des Anspruchs 4. Demnach ist die innere, d.h. die dem Boden des Aufnahmeraums bzw. die auf das stabartige Verbindungselement einwirkende Seite der Klemmschraube konvex gewölbt oder kegelförmig ausgebildet, wobei im letztgenannten Fall die Kegelspitze vorzugsweise abgerundet ist. Auf diese Weise ist eine punktförmige Beaufschlagung des stabartigen Verbindungselements erreicht, wodurch zugleich eine großflächige Anlage der Klemmschraube an dem Außenumfang des stabartigen Verbindungselements vermieden ist. Die Klemmschraube dient vornehmlich der Festlegung des stabartigen Verbindungselements in dem Aufnahmeraum des Haltemittels, ohne das stabartige Verbindungselement gegenüber der Klemmschraube auszurichten. Aufgrund dessen ist es möglich, daß das stabartige Verbindungselement in dem Aufnahmeraum des Haltemittels ohne weiteres auch in einer Richtung, die von der Senkrechten gegenüber der Längsachse des Haltemittels und/oder der Klemmschraube abweicht, fixierbar ist.

Um die erwähnte Verschwenkbarkeit und gleichzeitig dauerhafte Fixierung des in den Aufnahmeraum des Haltemittels eingebrachten Schwenklagerelements zu erhalten, sind die Merkmale des Anspruchs 5 von Vorteil. Danach erfolgt die Fixierung des Schwenklagerelements am Boden des Aufnahmeraums durch einen über dessen dem Boden des Aufnahmeraums zugewandte Lagerfläche vorstehenden Stift, der mit geringer Pressung in einer im Boden des Aufnahmeraums ausgebildeten Aufnahmenut gehalten ist, wobei die Aufnahmenut sich parallel zur Durchgangsebene des Aufnahmeraums erstreckt.

Alternativ erfolgt die Fixierung des Schwenklagerelements am Boden des Aufnahmeraums entsprechend Anspruch 6 durch einen über diesen vorstehenden Stift, der mit geringer Pressung in einer an der dem Boden des Aufnahmeraums zugewandten Lagerfläche des Schwenklagerelements ausgebildeten, vorzugsweise durchgehend ausgebildeten Aufnahmenut gehalten ist, wobei sich die Aufnahmenut parallel zur Aufnahmenut für das stabartige Verbindungselement erstreckt.

Weiter alternativ und bevorzugt erfolgt die Fixierung des Schwenklagerelements am Boden des Aufnahmeraums gemäß den Merkmalen des Anspruchs 7 durch einen Haltestift mit proximal angeordnetem Kopf, der mit seinem distalen Ende voran mit geringem Spiel durch einen sich parallel zur Aufnahmenut für das stabartige Verbindungselement erstreckenden Schlitz im Schwenklagerelement hindurch in eine korrespondierende Aufnahmebohrung am Boden des Aufnahmeraums hineinpreßbar ist, bis sein Kopf innerhalb einer in der Aufnahmenut für das stabartige Verbindungselement parallel zum vorgenannten Schlitz ausgebildeten Kopfnut zur Anlage kommt.

In weiterer Ausgestaltung der Erfindung liegt der Kopf des Haltestifts nach Anspruch 8 im montierten Zustand versenkt innerhalb der zugeordneten Kopfnut. Auf diese Weise ist sichergestellt, daß beim Verschwenken des Schwenklagerelements in einer Ebene parallel zur Ebene des im Schwenklagerelement ausgebildeten Schlitzes eine Kollision zwischen Kopf und stabartigem Verbindungselement ausgeschlossen ist.

Nach den Merkmalen des Anspruchs 9 ist der Haltestift mit seinem distalen Ende in der Aufnahmebohrung am Boden des Aufnahmeraums mit Preßsitz oder starkem Schiebesitz gehalten. Auf diese Weise ist eine dauerhafte, zudem einfache und damit kostengünstige Befestigung des Haltestiftes und damit des Schwenklagerelements insgesamt am Boden des Aufnahmeraums des Haltemittels gewährleistet.

Weiterhin liegt es gemäß Anspruch 10 im Rahmen der Erfindung, wenigstens ein Haltemittel als Pedikelschraube mit einem, insbesondere selbstschneidenden, Gewinde auszubilden, wodurch die Montage vereinfacht und zugleich eine hohe Kraftübertragung ermöglicht wird.

In bevorzugter Ausgestaltung der Erfindung ist die Pedikelschraube nach den Maßnahmen des Anspruches 11 im Bereich der ersten Gewindegänge des Gewindes zur Spanabhebung des Knochenmaterials des Wirbelsäulensegments längsgeschlitzt. Auf diese Weise wird das Knochenlager beim Eindrehen des Haltemittels bzw. der Pedikelschraube in den Wirbel durch Spanabhebung aufbereitet. Die entstehenden Knochenspähne verklemmen sich in den nachfolgenden Gewindegängen des Gewindes und verhindern somit zusätzlich eine selbsttätige Lösung des Haltemittels infolge einer erhöhten Schwergängigkeit.

Die Pedikelschraube ist zwischen dem Aufnahmeraum und dem Gewinde gemäß Anspruch 12 mit einem kegelstumpfartigen, sich zum Gewinde hin verjüngenden Abschnitt versehen, der paßgenau in eine entsprechend vorgefräste Ausnehmung im hochfesten Knochenmaterial (Corticalis) des Pedikels zur Anlage kommt. Die flächige Anlage dieses Abschnittes in der hochfesten Corticalis gewährleistet eine gute Übertragung hoher, auf das Haltemittel einwirkender Scherkräfte.

Nach den Maßnahmen des Anspruches 13 lassen sich auf den kegelstumpfartigen Abschnitt Distanzringe, Distanzhülsen etc. unterschiedlicher axialer Breite aufsetzen, so daß eine Implantation von benachbarten Haltemitteln auf jeweils gleichem Niveau möglich ist. Auf diese Weise wird die nachfolgende Einbringung des im wesentlichen stabförmigen Verbindungselementes erheblich erleichtert.

In alternativer Ausgestaltung des Haltemittels nach Anspruch 10 kann wenigstens ein Haltemittel auch entsprechend den Maßnahmen nach Anspruch 14 in ebenso vorteilhafter Weise als Haken zur Verankerung des Haltemittels am Pedikel, unter dem Gelenkfortsatz und am Querfortsatz ausgebildet sein. Die Form des Hakens selbst entspricht dabei bisher bekannt gewordenen, bewährten Haken.

Bei dem Erfordernis, einen Wirbelsäulenabschnitt besonders starr auszubilden, ist das Verbindungselement gemäß Anspruch 15 als biegesteifer Stab ausgebildet.

Zum Erhalt punktueller Bewegungsfreiheitsgrade kann innerhalb des durch ein Verbindungselement in Form eines biegesteifen Stabes ausgesteiften Wirbelsäulenabschnittes gemäß Anspruch 16 wenigstens ein Universalgelenk zwischen zwei Haltemitteln angeordnet werden. In näherer Ausgestaltung dieser Ausführungsform ist das Universalgelenk entsprechend Anspruch 17 zwischen zwei Teilen des Verbindungselements angeordnet. Dabei umfaßt das Universalgelenk ein etwa axial ausgerichtetes und topfförmiges Aufnahmeelement an einem Teil des Verbindungselements und eine endseitig an dem anderen Teil des Verbindungselements angebrachte, von dem topfförmigen Aufnahmeelement aufnehmbare und scheibenförmig abgerundete, kugelförmige od. dgl. flanschartige Verdickung. Insbesondere ist die Verdickung nach Anspruch 18 mit dem anderen Teil des Verbindungselementes über einen Gewindering, Sprengring oder dergleichen in dem topfförmigen Aufnahmeelement des einen Teils des Verbindungselements gehalten.

Weiterhin ist das Universalgelenk entsprechend Anspruch 19 vorteilhafterweise derart ausgebildet, daß die beiden Teile des Verbindungselements relativ zueinander in Axialrichtung unter elastischer Vorspannung bewegbar sind. Somit erlaubt das Universalgelenk nicht nur eine dreidimensionale Rotationsbewegung, sondern zusätzlich noch eine weitere Translationsbewegung.

Vorzugsweise ist nach Anspruch 20 im Aufnahmeelement des Universalgelenks eine (Druck-)Schraubenfeder angeordnet, die sich zwischen der Verdickung und dem am Aufnahmeelement befestigten Gewindering od.dgl. abstützt.

In alternativer Ausgestaltung des Verbindungselements als biegesteifer Stab kann das Verbindungselement ebensogut entsprechend Anspruch 21 in Form eines verbiegbares Drahts, flexiblen Kabels od. dgl. ausgestaltet sein. Auf diese Weise kann abhängig von der aufgebrachten Längsspannung des Drahtes, Kabels oder dergleichen zwischen zwei Haltemitteln eine individuell eingestellte, elastische, d. h. dynamische, Verbindung erhalten werden. Die Verwendung eines solchen Verbindungselements bietet sich beispielsweise bei der Behandlung chronischer Schmerzzustände im Lendenwirbelsäulenbereich oder auch für die Knochenneubildung im Spondylodesebezirk an.

Darüber hinaus liegt es nach Anspruch 22 im Rahmen der Erfindung, das Schwenklagerelement und/oder die Haltemittel und/oder die Klemmschraube und/oder das Verbindungselement aus Titan, einer hochfesten Titanlegierung od.dgl. körperverträglichem Material auszubilden. Hierdurch lassen sich Verschleiß, Korrosion und Bruch minimieren und eine ausgesprochen elastische, d. h. dynamische, Montage wegen des niedrigen E-Moduls dieser Legierungen ermöglichen.

Weitere Merkmale, Vorteile und Einzelheiten der Erfindung ergeben sich aus der nachfolgenden Beschreibung einiger bevorzugter Ausführungsformen der Erfindung sowie anhand der Zeichnungen. Hierbei zeigen:
- Fig. 1: eine Seitenansicht einer Ausführungsform eines erfindungsgemäß ausgebildeten Systems;
- Fig. 2: einen teilweisen Mittellängsschnitt durch eine Ausführung eines erfindungsgemäß ausgebildeten Haltemittels mit Schwenklagerelement und Verbindungselement in montiertem Zustand und in vergrößertem Maßstab;
- Fig. 3: einen teilweisen Mittellängsschnitt durch die Ausführung des Haltemittels gemäß Fig. 2 ohne Schwenklagerelement und Verbindungselement in unmontiertem Zustand und in vergrößertem Maßstab;
- Fig. 4: eine Vorder- bzw. Rückansicht auf das Schwenklagerelement nach Fig. 2 in vergrößertem Maßstab;
- Fig. 5: eine Draufsicht auf das Schwenklagerelement nach Fig. 2 in vergrößertem Maßstab;
- Fig. 6: einen Mittellängsschnitt durch das Schwenklagerelement nach Fig. 2 in vergrößertem Maßstab längs Linie VI-VI in Fig. 5;
- Fig. 7A und 7B: eine Seitenansicht und eine Draufsicht auf einen Haltestift für das Schwenklagerelement nach Fig. 2 in vergrößertem Maßstab;
- Fig. 8A bis 8D: Seitenansichten von erfindungsgemäß vorgesehenen Distanzringen und Distanzhülsen mit unterschiedlicher axialer Breite;
- Fig. 9: eine Explosionsdarstellung eines Verbindungselements mit einem Universalgelenk;

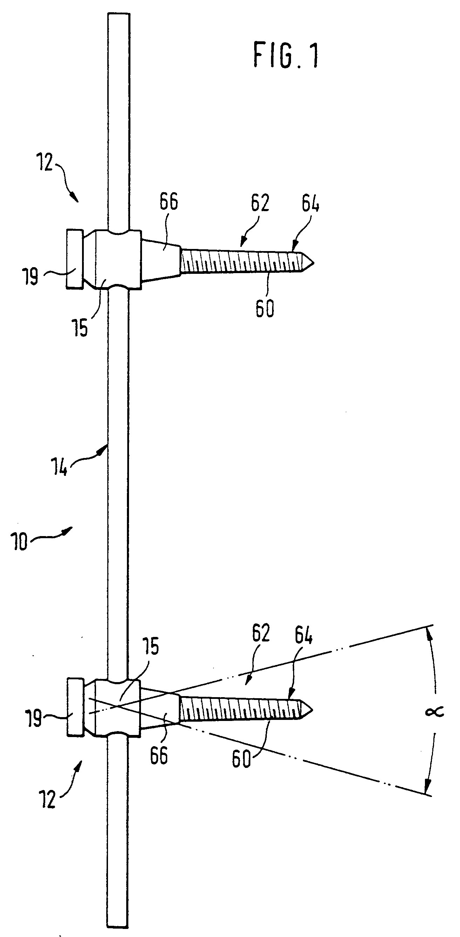

In Fig. 1 ist eine Ausführungsform eines Systems 10 zur Osteosynthese an der Wirbelsäule, insbesondere zur Stabilisierung von wenigstens zwei Wirbelsäulensegmenten (nicht gezeigt), dargestellt. Das System 10 umfaßt zwei Haltemittel 12, die jeweils an einem Wirbelsäulensegment der Wirbelsäule verankerbar sind.

Das Haltemittel 12 ist hier als Pedikelschraube ausgebildet. Diese wird in bekannter Weise in den Pedikel, d.h. in den Wirbelbogen zwischen Dornfortsatz, Querfortsatz und dem entsprechenden oberen Gelenkfortsatz eingeschraubt. In alternativer Ausgestaltung kann das Haltemittel 12 jedoch ebenso gut hakenförmig ausgebildet sein (nicht gezeigt). In diesem Fall wird das Haltemittel 12 am Pedikel, unter dem Gelenkfortsatz und Querfortsatz in an sich bekannter Weise verankert bzw. eingehängt.

Die beiden Haltemittel 12 entsprechend Fig. 1 sind zur Aufnahme und Festlegung eines die beiden Haltemittel 12 miteinander verbindenden, stabartigen Verbindungselements 14 vorgesehen. Je nach Erfordernis, d. h. an die jeweilige Indikation angepaßt, ist das Verbindungselement 14 entweder aus einem biegesteifen bzw. weitgehend biegesteifen Stab oder aus einem verbiegbaren Draht, flexiblen Kabel od.dgl. biegeelastischem Element hergestellt. Als Material für ein solches stabartiges Verbindungselement 14 bietet sich Titan, eine Titanlegierung od.dgl. körperverträgliches Material an.

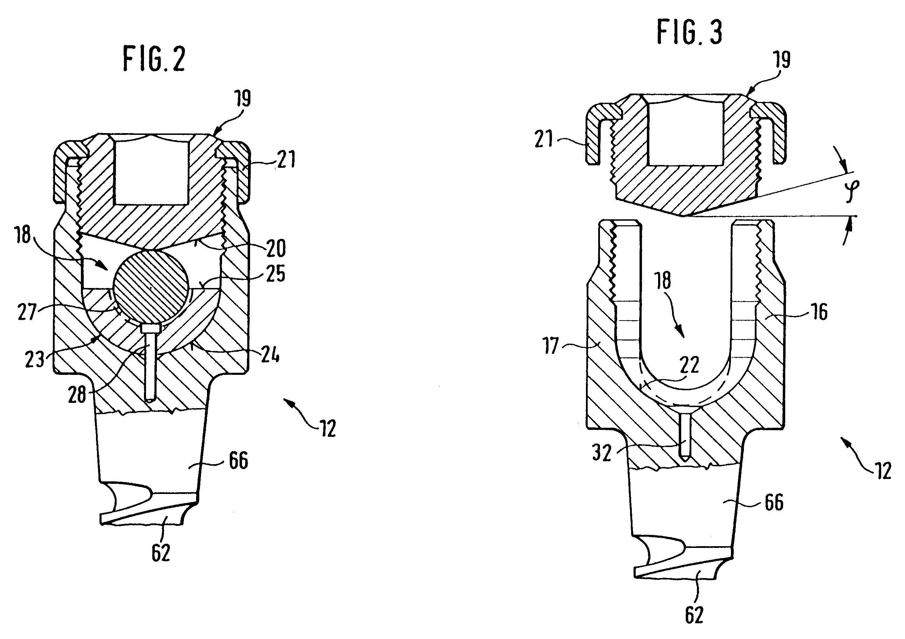

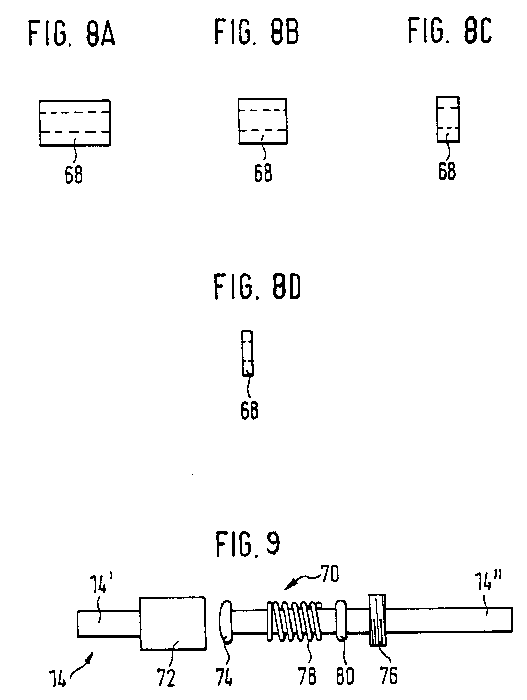

Entsprechend den Fig. 2 und 8 ist das Haltemittel 12 mit einem stimmgabelartigen Kopf 15 versehen, dessen beide Schenkel 16, 17 einen etwa U-förmigen Aufnahmeraum 18 für das Verbindungselement 14 begrenzen. Eine Klemmschraube 19 zur Festlegung des Verbindungselements 14 zwischen den beiden Schenkeln 16, 17 des stimmgabelartigen Schraubenkopfes 15 ist an dem Haltemittel 12 über eine Gewindeverbindung anbringbar, d.h. in den Aufnahmeraum 18 hineinschraubbar.

Die Klemmschraube 19 weist, wie in den Fig. 2 und 3 deutlich gezeigt, weiterhin einen etwa topfähnlichen Außenrand 21 auf, der die stirnseitigen Enden der beiden Schenkel 16, 17 des stimmgabelartigen Schraubenkopfes 15 übergreift und diese im montierten Zustand zusammenhält.

Der Boden 22 des Aufnahmeraums 18 ist entsprechend den Fig. 2 und 8 etwa konkav ausgebildet, und zwar in Zuordnung zu einem Schwenklagerelement 23, welches zwischen dem Boden 22 des Aufnahmeraums 18 und dem Verbindungselement 14 angeordnet ist. Die Lagerfläche 24 des Schwenklagerelements 23, die dem Boden 22 des Aufnahmeraums 18 zugewandt ist, ist komplementär konvex ausgestaltet.

Die Fig. 2 bis 6 zeigen eine erste Ausführungsform des Schwenklagerelements 23. Bei dieser Ausführungsform ist die Lagerfläche 24, die dem Boden 22 des Aufnahmeraums 18 zugewandt ist, schalenförmig, insbesondere hemisphärisch ausgebildet. Zudem weist die gegenüberliegende Seite 25 des Schwenklagerelements 23, die im montierten Zustand der Klemmschraube 19 zugewandt ist, eine Nut 26 zur Aufnahme des stabartigen Verbindungselements 14 auf.

Die Nut 26 zur Aufnahme des stabartigen Verbindungselements 14, die im Schwenklagerelement 23 ausgebildet ist, weist einen etwa halbkreisförmigen Querschnitt entsprechend dem Querschnitt des Verbindungselements 14 auf. Weiterhin sind in die Oberfläche der Nut 26 Querrippen 27, insbesondere ein Feingewinde, eingearbeitet. Die Querrippen 27 wirken einer axialen Verschiebung des Verbindungselements 14 nach dessen Montage in dem etwa U-förmigen Aufnahmeraum 18 des Haltemittels 12 längs der Durchgangsebene des Aufnahmeraums 18 zusätzlich entgegen.

Die innere Seite 20 der Klemmschraube 19, die auf das stabartige Verbindungselement 14 einwirkt, ist konvex gewölbt oder kegelförmig ausgebildet. Vorzugsweise beträgt der Winkel ϕ der Kegelfläche (vgl. Fig. 3) etwa 5° bis 35°, insbesondere ca. 20°. Im Falle einer kegelförmigen Ausbildung der inneren Seite 20 der Klemmschraube 19 ist dabei deren Kegelspitze vorzugsweise abgerundet.

Das Schwenklagerelement 23 ist am Boden 22 des Aufnahmeraums 18 derart gehalten, daß es sowohl in einer Ebene parallel zur Durchgangsebene des Aufnahmeraums 18 als auch senkrecht dazu verschwenkbar ist. Zu diesem Zweck weist das Schwenklagerelement 23 eine halbschalenförmige, insbesondere hemisphärische Lagerfläche 24 auf entsprechend der sphärischen Ausbildung des Bodens 22 des Aufnahmeraums 18.

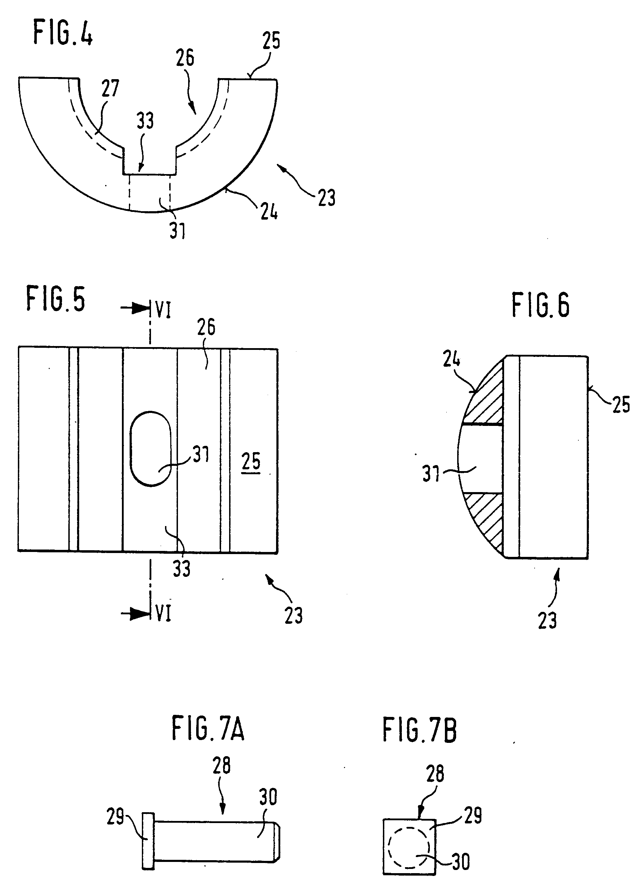

Die Fixierung des Schwenklagerelements 23 am Boden 22 des Aufnahmeraums 18 erfolgt durch einen wie in den Fig. 7A und 7B gezeigten Haltestift 28 mit einem proximal angeordneten Kopf 29 und einem distalen Ende 30. Der Haltestift 28 erstreckt sich mit seinem distalen Ende 30 mit geringem Spiel durch einen Schlitz 31 im Schwenklagerelement 23, welcher parallel zur Aufnahmenut 26 für das stabartige Verbindungselement 14 verläuft, hindurch und ist in eine korrespondierende Aufnahmebohrung 32 am Boden 22 des Aufnahmeraums 18 hineinpreßbar. Der Kopf 29 des Haltestifts 28 kommt dabei innerhalb einer Kopfnut 33 zur Anlage, die in der Aufnahmenut 26 für das stabartige Verbindungselement 14 parallel zum vorgenannten Schlitz 31 ausgebildet ist. Der Kopf 29 des Haltestifts 28 liegt somit im montierten Zustand versenkt innerhalb der zugeordneten Kopfnut 33, so daß beim Verschwenken des Schwenklagerelements 23 in einer Ebene parallel zur Ebene des im Schwenklagerelement 23 ausgebildeten Schlitzes 31 keine Kollision zwischen dem Kopf 29 und dem stabartigen Verbindungselement 14 auftritt.

Der Haltestift 28 ist mit seinem distalen Ende 30 in der Aufnahmebohrung 32 am Boden 22 des Aufnahmeraums 18 mit Preßsitz, starkem Schiebesitz oder dergleichen gehalten.

In alternativer nicht im einzelnen dargestellten Ausgestaltung kann die Fixierung des Schwenklagerelements 23 am Boden 22 des Aufnahmeraums 18 auch durch einen über dessen dem Boden 22 des Aufnahmeraums 18 zugewandte Lagerfläche 24 vorstehenden Stift erfolgen, der mit geringer Pressung in einer im Boden 22 des Aufnahmeraums 18 ausgebildeten Aufnahmenut (nicht dargestellt) gehalten ist, wobei sich die Aufnahmenut parallel zur Durchgangsebene des Aufnahmeraums 18 erstreckt. Ebenso ist es in einer weiteren alternativen Ausgestaltung denkbar, daß die Fixierung des Schwenklagerelements 23 am Boden 22 des Aufnahmeraums 18 durch einen über diesen vorstehenden Stift erfolgt, der mit geringer Pressung in einer an der dem Boden 22 des Aufnahmeraums 18 zugewandten Lagerfläche 24 des Schwenklagerelements 23 ausgebildeten, insbesondere durchgehend ausgebildeten Aufnahmenut gehalten ist, wobei sich die Aufnahmenut parallel zur Aufnahmenut 26 für das stabartige Verbindungselement 14 erstreckt.

Entsprechend Fig. 1 und 2 ist das Haltemittel 12 als Pedikelschraube mit einem Abschnitt 60 mit selbstschneidendem Gewinde 62 ausgebildet. Das Gewinde 62 ist im Bereich 64 der ersten Gewindegänge zur Spanabhebung des Knochenmaterials des Wirbels längsgeschlitzt.

Zwischen dem Kopf 15 der Pedikelschaube und dem Gewinde 62 ist ein Abschnitt 66 ausgebildet, der sich in Richtung zur Schraubenspitze hin kegelstumpfförmig verjüngt. Der Abschnitt 66 dient einer paßgenauen Anlage in einer entsprechend vorgefrästen Ausnehmung im Knochenmaterial des Wirbels, um auf diese Weise auftretende, zumeist sehr hohe Scherkräfte abzufangen. Zusätzlich können auf den kegelstumpfförmigen Abschnitt 66 in Fig. 8 A bis 8 D beispielhaft dargestellte Distanzringe 68 unterschiedlicher axialer Breite aufgesetzt werden.

Wie in Fig. 9 gezeigt, ist das als biegesteifer Stab ausgebildete Verbindungselement 14 zusätzlich mit einem Universalgelenk 70 versehen, das zwischen zwei Haltemitteln 12 (in Fig. 5 nicht dargestellt) angeordnet ist. Das Universalgelenk 70 verbindet zwei Teile 14', 14'' des Verbindungselements 14.

Das Universalgelenk 70 umfaßt entsprechend Fig. 9 ein etwa axial ausgerichtetes topfförmiges Aufnahmeelement 72, das mit dem einen Teil 14' des Verbindungselements 14 starr verbunden ist. Des weiteren umfaßt das Universalgelenk 70 eine Verdickung 74, die endseitig an dem anderen Teil 14'' des Verbindungselements 14 angebracht ist. Diese wird von dem topfförmigen Aufnahmeelement 72 aufgenommen. Im vorliegenden Fall ist die Verdickung 74 scheibenförmig abgerundet bzw. flanschartig ausgebildet. Die Verdickung 74 ist mit dem anderen Teil 14'' des Verbindungselements 14 über einen Gewindering 76 in dem topfförmigen Aufnahmeelement 72 des einen Teils 14' des Verbindungselements 14 gehalten.

Das Universalgelenk 70 ist darüber hinaus derart ausgebildet, daß die beiden Teile 14', 14'' des Verbindungselementes 14 nicht nur eine seitliche Drehbewegung bzw. Abknickbewegung relativ zueinander, sondern auch eine Translationsbewegung in Axialrichtung vollziehen können. Vorzugsweise erfolgt eine solche Translationsbewegung in Axialrichtung unter Einwirkung von Federkräften. Demnach ist in dem Aufnahmeelement 72 des Universalgelenks 70 eine (Druck-)Schraubenfeder 78 angeordnet, die sich zwischen der Verdickung 74 des anderen Teils 14'' des Verbindungselements 14 und dem an dem Aufnahmeelement 72 des einen Teils 14' des Verbindungselements 14 befestigten Gewindering 76 unter Zwischenschaltung einer Beilagscheibe 70 abstützt. Durch die Wahl der Länge des topfförmigen Aufnahmeelementes 72 sowie Dimensionierung der Schraubenfeder 78 läßt sich die Translationsbewegung der beiden Teile 14', 14'' des Verbindungselements 14, jeweils an die spezielle Anwendung angepaßt, beliebig variieren.

## Patentansprüche

1. System zur Osteosynthese an der Wirbelsäule, insbesondere zur Stabilisierung von Wirbelsäulensegmenten, bestehend aus
- mindestens einem stabartigen Verbindungselement (14),
- mindestens zwei, jeweils an einem Wirbelsäulensegment verankerbaren Haltemitteln (12) mit einem stimmgabelartigen Kopf (15), dessen beide Schenkel (16, 17) einen etwa U-förmigen Aufnahmeraum (18) für das Verbindungselement (14) begrenzen,
- einer Klemmschraube (19), die zur Festlegung des Verbindungselements (14) zwischen den beiden Schenkeln (16, 17) des stimmgabelartigen Schraubenkopfes (15) in den Aufnahmeraum (18) hineinschraubbar ist, wobei
- der Boden (22) des Aufnahmeraums (18) konkav schalenförmig ausgebildet ist in Zuordnung zu einem zwischen dem Boden (22) des Aufnahmeraums (18) und dem Verbindungselement (14) angeordneten Schwenklagerelement (23), dessen dem Boden (22) des Aufnahmeraums (18) zugewandte Lagerfläche (24) komplementär konvex ausgebildet ist,
**dadurch gekennzeichnet**,
daß das Schwenklagerelement (23) mit einer dem Boden (22) des Aufnahmeraums (18) zugewandten, hemisphärisch ausgebildeten Lagerfläche (24) und mit einer klemmschraubenseitig offenen Nut (26) zur Aufnahme des stabartigen Verbindungselements (14) versehen ist, und das Schwenklagerelement (23) am Boden (22) des Aufnahmeraums (18) derart gehalten ist, daß es sowohl in einer Ebene parallel zur Durchgangsebene des Aufnahmeraums (18) als auch senkrecht dazu verschwenkbar ist.

2. System nach Anspruch 1,
dadurch gekennzeichnet,
daß die im Schwenklagerelement (23) ausgebildete Nut (26) zur Aufnahme des stabartigen Verbindungselements (14) entsprechend dessen Querschnitt einen etwa halbkreisförmigen Querschnitt aufweist.

3. System nach Anspruch 2,
dadurch gekennzeichnet,
daß in die Oberfläche der Nut (26) Querrippen (27), insbesondere ein Feingewinde, eingearbeitet sind.

4. System nach einem der Ansprüche 1 bis 3,
dadurch gekennzeichnet,
daß die innere, d.h. die dem Boden (22) des Aufnahmeraums (18) bzw. die auf das stabartige Verbindungselement (14) einwirkende Seite (20) der Klemmschraube (19) konvex gewölbt oder kegelförmig ausgebildet ist, wobei im letztgenannten Fall die Kegelspitze vorzugsweise abgerundet ist.

5. System nach einem der Ansprüche 1-4,
dadurch gekennzeichnet,
daß die Fixierung des Schwenklagerelements (23) am Boden (22) des Aufnahmeraums (18) durch einen über dessen dem Boden (22) des Aufnahmeraums (18) zugewandte Lagerfläche (24) vorstehenden Stift erfolgt, der mit geringer Pressung in einer im Boden (22) des Aufnahmeraums (18) ausgebildeten Aufnahmenut gehalten ist, wobei die Aufnahmenut sich parallel zur Durchgangsebene des Aufnahmeraums (18) erstreckt.

6. System nach einem der Ansprüche 1-4,
dadurch gekennzeichnet,
daß die Fixierung des Schwenklagerelements (23) am Boden (22) des Aufnahmeraums (18) durch einen über diesen vorstehenden Stift erfolgt, der mit geringer Pressung in einer an der dem Boden (22) des Aufnahmeraums (18) zugewandten Lagerfläche (24) des Schwenklagerelements (23) ausgebildeten, insbesondere durchgehend ausgebildeten Aufnahmenut gehalten ist, wobei sich die Aufnahmenut parallel zur Aufnahmenut (26) für das stabartige Verbindungselement (14) erstreckt.

7. System nach einem der Ansprüche 1-4,
dadurch gekennzeichnet,
daß die Fixierung des Schwenklagerelements (23) am Boden (22) des Aufnahmeraums (18) durch einen Haltestift (28) mit proximal angeordnetem Kopf (29) erfolgt, der mit seinem distalen Ende (30) mit geringem Spiel durch einen sich parallel zur Aufnahmenut (26) für das stabartige Verbindungselement (14) erstreckenden Schlitz (31) im Schwenklagerelement (23) hindurch in eine korrespondierende Aufnahmebohrung (32) am Boden (22) des Aufnahmeraums (18) hineinpreßbar ist, bis sein Kopf (29) innerhalb einer in der Aufnahmenut (26) für das stabartige Verbindungselement (14) parallel zum vorgenannten Schlitz (31) ausgebildeten Kopfnut (33) zur Anlage kommt.

8. System nach Anspruch 7,
dadurch gekennzeichnet,
daß der Kopf (29) des Haltestifts (28) im montierten Zustand versenkt innerhalb der zugeordneten Kopfnut (33) liegt, so daß beim Verschwenken des Schwenklagerelements (23) in einer Ebene parallel zur Ebene des im Schwenklagerelement (23) ausgebildeten Schlitzes (31) keine Kollision zwischen Kopf (29) und stabartigem Verbindungselement (14) auftritt.

9. System nach Anspruch 7 oder 8,
dadurch gekennzeichnet,
daß der Haltestift (28) mit seinem distalen Ende (30) in der Aufnahmebohrung (32) am Boden (22) des Aufnahmeraums (18) mit Preßsitz oder starkem Schiebesitz gehalten ist.

10. System nach wenigstens einem der Ansprüche 1 bis 9,
dadurch gekennzeichnet,
daß wenigstens ein Haltemittel (12) als Pedikelschraube mit einem, insbesondere selbstschneidenden, Gewinde (62) ausgebildet ist.

11. System nach Anspruch 10,
dadurch gekennzeichnet,
daß die Pedikelschraube im Bereich (64) der ersten Gewindegänge des Gewindes (62) zur Spanabhebung des Knochenmaterials des Wirbelsäulensegments längsgeschlitzt ist.

12. System nach Anspruch 10 und/oder 11,
dadurch gekennzeichnet,
daß die Pedikelschraube zwischen dem Kopf (15) und dem Gewinde (62) einen sich in Richtung zum Gewinde (62) hin kegelstumpfartig verjüngenden Abschnitt (66) zur paßgenauen Anlage in einer entsprechend vorgefrästen Ausnehmung im Knochen des Wirbelsäulensegments aufweist.

13. System nach Anspruch 12,
dadurch gekennzeichnet,
daß auf den kegelstumpfartigen Abschnitt (66) der Pedikelschraube Distanzringe (68) unterschiedlicher axialer Breite aufsetzbar sind.

14. System nach wenigstens einem der Ansprüche 1 bis 13,
dadurch gekennzeichnet,
daß wenigstens ein Haltemittel (12) als Haken zur Verankerung am Pedikel, unter dem Gelenkfortsatz und am Querfortsatz ausgebildet ist.

15. System nach wenigstens einem der Ansprüche 1 bis 14,
dadurch gekennzeichnet,
daß das Verbindungselement (14) als biegesteifer Stab oder dergleichen ausgebildet ist.

16. System nach Anspruch 15,
dadurch gekennzeichnet,
daß das als biegesteifer Stab ausgebildete Verbindungselement (14) wenigstens ein zwischen zwei Haltemitteln (12) angeordnetes Universalgelenk (70) umfaßt.

17. System nach Anspruch 16,
dadurch gekennzeichnet,
daß das Universalgelenk (70) zwischen zwei Teilen (14', 14'') des Verbindungselements (14) angeordnet ist und ein sich axial erstreckendes topfförmiges Aufnahmeelement an dem einen Teil (14') des Verbindungselements (14) und eine endseitig an dem anderen Teil (14'') des Verbindungselements (14) angebrachte, von dem topfförmigen Aufnahmeelement (72) aufnehmbare und scheibenförmig abgerundete, kugelförmige oder dergleichen flanschartige Verdickung (74) umfaßt.

18. System nach Anspruch 17,
dadurch gekennzeichnet,
daß die Verdickung (74) mit dem anderen Teil (14'') des Verbindungselements (14) durch einen Gewindering (76), Sprengring oder dergleichen in dem topfförmigen Aufnahmeelement (72) des einen Teils (14') des Verbindungselements (14) gehalten ist.

19. System nach Anspruch 17 und/oder 18,
dadurch gekennzeichnet,
daß das Universalgelenk (70) derart ausgebildet ist, daß die beiden Teile (14', 14'') des Verbindungselements (14) relativ zueinander in Axialrichtung, insbesondere unter elastischer Vorspannung, bewegbar sind.

20. System nach Anspruch 19,
dadurch gekennzeichnet,
daß in dem topfförmigen Aufnahmeelement (72) des Universalgelenks (70) eine (Druck-)Schraubenfeder (78) oder dergleichen angeordnet ist, die sich zwischen der Verdickung (74) und dem an dem Aufnahmeelement (72) befestigten Gewindering (76), Sprengring oder dergleichen abstützt.

21. System nach wenigstens einem der Ansprüche 1 bis 14,
dadurch gekennzeichnet,
daß das Verbindungselement (14) als verbiegbarer Draht oder in Form eines flexiblen Kabels ausgebildet ist.

22. System nach wenigstens einem der Ansprüche 1 bis 21,
dadurch gekennzeichnet,
daß das Schwenklagerelement (23) und/oder das Haltemittel (12) und/oder die Klemmschraube (19) und/oder das Verbindungselement (14) aus Titan, einer Titanlegierung oder dergleichen nicht-rostendem Material besteht/bestehen.

## Claims

1. System for osteosynthesis in the vertebral column, particularly for stabilising vertebral segments, comprising
- at least one rod-like connecting member (14),
- at least two retaining means (12) each anchorable on a vertebral segment, with a head (15) in the form of a tuning fork, whose two legs (16, 17) define a roughly U-shaped receiving cavity (18) for the connecting member (14),
- a clamp screw (19) which may be screwed into the receiving cavity (18) in order to secure the connecting member (14) between both legs (16, 17) of the tuning-fork-like screw head (15), the base (22) of the receiving cavity (18) being in a concave shell shape, in association with a pivot bearing member (23) disposed between the base (22) of the receiving cavity (18) and the connecting member (14), the bearing face (24) of said pivot bearing member facing the base (22) of the receiving cavity (18) being in a complementary convex shape,
characterised in that
the pivot bearing member (23) is provided with a hemispherically-shaped bearing face (24) facing the base (22) of the receiving cavity, and with a groove (26), open on the clamp screw side, for receiving the rod-like connecting member (14), and the pivot bearing member (23) is held on the base (22) of the receiving cavity (18) in such a way that it is pivotable both in a plane parallel to the passage plane of the receiving cavity (18) and also vertically thereto.

2. System according to claim 1,
characterised in that
the groove (26) formed in the pivot bearing member (23) in order to receive the rod-like connecting member (14) has, corresponding with the cross-section of the latter, a roughly semicircular cross-section.

3. System according to claim 2,
characterised in that
transverse ribs (27), in particular a fine threading, are machined into the surface of the groove (26).

4. System according to one of claims 1 to 3,
characterised in that
the inner side (20) of the clamp screw (19), i.e. the side facing the base (22) of the receiving cavity (18) or acting on the rod-shaped connecting member (14), has a convex bulge or is conical in shape, the point of the cone in the latter case being rounded off.

5. System according to one of claims 1 to 4,
characterised in that
the pivot bearing member (23) is secured on the base (22) of the receiving cavity (18) by a pin projecting over its bearing face (24) facing the base (22) of the receiving cavity (18), said pin being held with a small degree of pressure in a receiving groove formed in the base (22) of the receiving cavity (18), the receiving groove extending parallel to the passage plane of the receiving cavity (18).

6. System according to one of claims 1 to 4,
characterised in that
the pivot bearing member (23) is secured on the base (22) of the receiving cavity (18) by a pin projecting over said receiving cavity and held with a small degree of pressure in a receiving groove, particularly continuous in form, formed on a bearing face (24) of the pivot bearing member (23) facing the base (22) of the receiving cavity (18), said receiving groove extending parallel to the receiving groove (26) for the rod-like connecting member (14).

7. System according to one of claims 1 to 4,
characterised in that
the pivot bearing member (23) is secured to the base (22) of the receiving cavity (18) by a retainer pin (28) with a proximally-disposed head (29) which can be pressed with its distal end (30) with a small degree of play through a slot (31) in the pivot bearing member (23) extending parallel to the receiving groove (26) for the rod-like connecting member (14), through into a corresponding receiving bore (32) on the base (22) of the receiving cavity 918), until its head (29) comes into contact inside a head groove (33) formed in the receiving groove (26) for the rod-like connecting member (14) parallel to the abovenamed slot (31).

8. System according to claim 7,
characterised in that
the head (29) of the retainer pin (28), in the assembled state, lies in a countersunk manner inside the associated head groove (33) so that, upon pivoting of the pivot bearing member (23) in a plane parallel to the plane of the slot (31) formed in the pivot bearing member (23), no collision occurs between head (29) and rod-like connecting member (14).

9. System according to claim 7 or 8,
characterised in that
the retainer pin (28) is held with its distal end (30) in the receiving bore (32) on the base (22) of the receiving cavity (18) with a push fit or with a tight sliding fit.

10. System according to at least one of claims 1 to 9,
characterised in that
at least one retaining means (12) is in the form of a stem screw with a thread (62), particularly a self-tapping thread.

11. System according to claim 10,
characterised in that
the stem screw is longitudinally slotted in the region (64) of the first thread turns of the thread (62) in order to provide chip-removing processing of the bone material of the vertebral segment.

12. System according to claim 10 or 11,
characterised in that
the stem screw has, between the head (15) and the thread (62), a portion (66) tapering frustoconically in the direction of the thread (62, for contact with a precise fit in a correspondingly pre-machined recess in the bone of the vertebral segment.

13. System according to claim 12,
characterised in that
spacer rings (668) of various axial widths can be set on the frustoconical portion (66) of the stem screw.

14. System according to at least one of claims 1 to 13,
characterised in that
at least one retaining means (12) is in the form of a hook for anchoring on the stem, under the joint process and on the transverse process.

15. System according to at least one of claims 1 to 14,
characterised in that
the connecting member (14) is in the form of a bending-resistant rod or the like.

16. System according to claim 15,
characterised in that
the connecting member (14) in the form of a bending-resistant rod includes at least one universal joint (70) disposed between two retaining means (12).

17. System according to claim 16,
characterised in that
the universal joint (70) is disposed between two portions (14', 14'') of the connecting member (14), and includes an axially-extending cup-shaped receiving member on one portion (14') of the connecting member (14), and a disc-like rounded spherical or similarly flange-like thickened portion (74) attached at the end on the other portion (14'') of the connecting member (14) and capable of being housed by the cup-shaped receiving member (72).

18. System according to claim 17,
characterised in that
the thickened portion (74) is held with the other portion (14'') of the connecting member (14) by a threaded ring (76), snap ring or the like in the cup-shaped receiving member (72) of one portion (14') of the connecting member (14).

19. System according to claim 17 and/or 18,
characterised in that
the universal joint (70) is so designed that both portions (14', 14'') of the connecting member (14) are movable relative to one another in the axial direction, particularly with resilient spring bias.

20. System according to claim 19,
characterised in that
there is disposed in the cup-shaped receiving member (72) of the universal joint (70) a (pressure) helical spring (78) or the like, which is supported between the thickened portion (74) and the threaded ring (76), snap ring or the like attached to the receiving member (72).

21. System according to at least one of claims 1 to 14,
characterised in that
the connecting member (14) is in the form of a bendable wire, or in the form of a flexible cable.

22. System according to at least one of claims 1 to 21,
characterised in that
the pivot bearing member (23) and/or the retainer means (12) and/or the clamp screw (19) and/or the connecting member (14) is/are made of titanium, a titanium alloy or such non-rusting material.

## Revendications

1. Dispositif pour ostéosynthèse sur la colonne vertébrale, en particulier pour la stabilisation des vertèbres, comprenant :
- au moins un élément de liaison (14) en forme de baguette,
- au moins deux moyens de fixation (12) susceptibles d'être chacun ancrés dans une vertèbre, ces moyens présentant une tête (15) de type en forme de fourche dont les deux branches (16,17) définissent un espace de réception (18) sensiblement en forme de U pour l'élément de liaison (14),
- une vis de serrage (19) susceptible d'être vissée dans l'espace de réception (18) pour fixer l'élément de liaison (14) entre les deux branches (16,17) de la tête de vis (15) en forme de fourche,
- tête dont le fond (22) de l'espace de réception (18) est façonné sous la forme d'une cuvette concave en correspondance avec un élément de coussinet de basculement (23) agencé entre le fond (22) de l'espace de réception (18) et l'élément de liaison (14), élément de coussinet dont la surface d'appui (24) en vis-à-vis du fond (22) de l'espace de réception (18) est de forme convexe complémentaire,
caractérisé en ce que
l'élément de coussinet de basculement (23) est prévu avec une surface d'appui (24) hémisphérique en correspondance avec le fond (22) de l'espace de réception (18), et est prévu avec une échancrure (26) ouverte du côté de la vis de serrage pour réception de l'élément de liaison en forme de baguette (14),
et en ce que l'élément de coussinet de basculement (23) est retenu contre le fond (22) de l'espace de réception (18) de telle sorte qu'il peut basculer aussi bien dans un plan parallèle au plan médian de l'espace de réception (18) que dans un plan perpendiculaire.

2. Dispositif selon la revendication 1, caractérisé en ce que l'échancrure (26) formée dans l'élément de coussinet de basculement (23) pour recevoir l'élément de liaison en forme de baguette (14) présente une section sensiblement semi-circulaire en correspondance avec la section de l'élément de liaison.

3. Dispositif selon la revendication 2, caractérisé en ce que des cannelures (27) sont façonnées dans la surface de l'échancrure (26), et plus particulièrement un filetage fin.

4. Dispositif selon l'une des revendications 1 à 3, caractérisé en ce que le côté interne (20) de la vis de serrage (19), c'est-à-dire orienté vers le fond (22) de l'espace de réception (18) et agissant respectivement contre l'élément de liaison en forme de baguette (14), présente un bombement convexe ou sphérique, la pointe de la sphère étant dans ce dernier cas avantageusement arrondie.

5. Dispositif selon l'une des revendications 1 à 4, caractérisé en ce que la fixation de l'élément de coussinet de basculement (23) au fond (22) de l'espace de réception (18) est réalisée au moyen d'une tige protubérant de la zone du fond (22) en correspondance avec la surface d'appui (24), laquelle tige est retenue avec une faible pression dans un orifice de réception ménagé dans le fond (22) de l'espace (18), cet orifice de réception s'étendant parallèlement au plan médian de l'espace de réception (18).

6. Dispositif selon l'une des revendications 1 à 4, caractérisé en ce que la fixation de l'élément de coussinet de basculement (23) au fond (22) de l'espace de réception (18) est réalisée au moyen d'une tige protubérant de ce fond, laquelle tige est tenue dans un orifice de réception ménagé dans la surface d'appui (24) de l'élément de coussinet de basculement (23) en correspondance avec le fond (22) de l'espace de réception (18), et en particulier un orifice de réception traversant, cet orifice s'étendant parallèlement à l'échancrure (26) de réception de l'élément de liaison en forme de baguette (14).

7. Dispositif selon l'une des revendications 1 à 4, caractérisé en ce que la fixation de l'élément de coussinet de basculement (23) au fond (22) de l'espace de réception (18) est réalisée au moyen d'une tige de retenue (28) présentant une tête (29) proximale, et dont la pointe distale (30) passe avec un faible jeu au travers d'une fente (31) de l'élément de coussinet de basculement (23), cette fente s'étendant parallèlement à l'échancrure (26) de réception de l'élément de liaison (14) en forme de baguette, cette pointe distale pouvant être pressée dans un perçage de réception correspondant (32) ménagé dans le fond (22) de l'espace de réception (18), et ceci jusqu'à ce que la tête (29) vienne en appui complètement à l'intérieur d'un logement de tête (33) ménagé dans l'échancrure (26) de réception de l'élément de liaison en forme de baguette (14) et ceci parallèlement à la fente (31) précitée.

8. Dispositif selon la revendication 7, caractérisé en ce que la tête (29) de la tige de retenue (28) est contenue, à l'état assemblé, à l'intérieur de son logement de tête (33) correspondant de telle sorte que, lors du basculement de l'élément de coussinet de basculement (23) dans un plan parallèle au plan de la fente ménagée (31) dans l'élément de coussinet (23), il ne se produise aucune collision entre cette tête (29) et l'élément de liaison en forme de baguette (14).

9. Dispositif selon la revendication 7 ou 8, caractérisé en ce que la tige de fixation (28) est retenue en son extrémité distale (30) dans le perçage de réception (32) du fond (22) de l'espace de réception (18) par compression ou coincement fort.

10. Dispositif selon l'une des revendications 1 à 9, caractérisé en ce qu'au moins l'un des moyens de fixation (12) se présente sous la forme d'une vis de corps de vertèbre avec un filetage (62), plus particulièrement un filetage autoperçant.

11. Dispositif selon la revendication 10, caractérisé en ce que la vis de corps de vertèbre est fendue longitudinalement dans la zone (64) des premiers tours du filetage (62) pour libérer la tension dans le matériau osseux des vertèbres.

12. Dispositif selon la revendication 10 et/ou 11, caractérisé en ce que la vis de corps de vertèbre présente entre la tête (15) et le filetage (62) une zone de rétrécissement (66) en forme de tronc de cône orientée vers le filetage (62) prévu pour se conformer exactement dans un fraisage correspondant ménagé préalablement dans l'os de la vertèbre.

13. Dispositif selon la revendication 12, caractérisé en ce que des bagues d'écartement (68) de différentes longueurs axiales peuvent être installées sur la zone en tronc de cône (66) de la vis de corps de vertèbre.

14. Dispositif selon l'une des revendications 1 à 13, caractérisé en ce qu'au moins l'un des moyens de fixation (12) est sous la forme d'un crochet pour ancrage au corps de vertèbre sous l'apophyse d'articulation ou aux apophyses transversales.

15. Dispositif selon l'une des revendications 1 à 14, caractérisé en ce que l'élément de liaison (14) est sous la forme d'une baguette rigide en flexion ou analogue.

16. Dispositif selon la revendication 15, caractérisé en ce que l'élément de liaison (14) sous la forme d'une baguette rigide en flexion comprend au moins une articulation universelle (70) agencée entre deux moyens de fixation (12).

17. Dispositif selon la revendication 16, caractérisé en ce que l'articulation universelle (70) est agencée entre deux parties (14',14'') de l'élément de liaison (14), et en ce qu'il comprend en l'une des parties (14') de l'élément de liaison (14) un élément femelle ayant la forme d'un réceptacle longitudinal ; et une surépaisseur (74) rapportée en l'extrémité de l'autre pièce (14'') de l'élément de liaison (14), cette surépaisseur ayant la forme d'un disque à tranche arrondie, ou d'une boule, ou d'une flasque analogue susceptible d'être insérée dans l'élément femelle (72) en forme de réceptacle.

18. Dispositif selon la revendication 17, caractérisé en ce que la surépaisseur (74) de l'autre partie (14'') de l'élément de liaison (14) est retenue dans l'élément femelle (72) en forme de réceptacle de la première partie (14') de l'élément de liaison (14) au moyen d'une bague filetée (76), d'une bague élastique ou analogue.

19. Dispositif selon la revendication 17 ou 18, caractérisé en ce que l'articulation universelle (70) est agencée de telle sorte que les deux parties (14',14'') de l'élément de liaison (14) sont mobiles axialement l'une par rapport à l'autre, et plus particulièrement sous tension élastique.

20. Dispositif selon la revendication 19, caractérisé en ce qu'un ressort hélicoïdal (de pression) (78) ou analogue est agencé dans l'élément femelle (72) en forme de réceptacle de l'articulation universelle (70), lequel ressort agit entre la surépaisseur (74) et la bague filetée (76), bague élastique ou analogue fixée dans l'élément femelle (72).

21. Dispositif selon l'une des revendications 1 à 14, caractérisé en ce que l'élément de liaison (14) se présente sous la forme d'un câble flexible ou d'un fil susceptible d'être plié.

22. Dispositif selon l'une des revendications 1 à 21, caractérisé en ce que l'élément de coussinet de basculement (23) et/ou les moyens de fixation (12) et/ou la vis de serrage (19) et/ou l'élément de liaison (14) sont réalisés en titane ou en un matériau recouvert de titane ou autre matériau inoxydable.
